# EUROPEAN PATENT SPECIFICATION

(11) **EP 2 858 494 B1**
(45) Date of publication and mention of the grant of the patent: **14.11.2018**
(21) Application number: 13804049.8
(22) Date of filing: 28.05.2013
(51) Int. Cl.: A01N 25/14, A01N 25/30, A01N 25/32, A01N 43/40, A01N 43/707, A61P 13/00

(54) **A HERBICIDAL COMPOSITION AND PROCESS THEREOF**
HERBIZIDE ZUSAMMENSETZUNG UND VERFAHREN DAFÜR
COMPOSITION HERBICIDE ET PROCÉDÉ POUR CELLE-CI

(30) Priority: 11.06.2012 IN 653KO2012
(43) Date of publication of application: 15.04.2015
(73) Proprietor: UPL Limited, Mumbai 400 052 MAH (IN)
(72) Inventor: SHROFF, Jaidev, Rajnikant, State of Maharashtra Mumbai 400 052 (IN); SHROFF, Vikram, Rajnikant, State of Maharashtra Mumbai 400 052 (IN); SHIRSAT, Rajan, Ramakant, State of Maharashtra Mumbai 400 052 (IN); KUMAR, Ajit, State of Maharashtra Mumbai 400 052 (IN)
(74) Representative: HGF Limited
(86) International application number: PCT/IB2013/054401
(87) International publication number: WO 2013/186652

(56) References cited:
- WO-A1-90/02486
- WO-A1-95/18531
- WO-A1-2009/113093
- WO-A2-2009/100101
- WO-A2-2012/009489
- DE-A1-102005 051 830
- US-A1- 2011 306 502
- US-B1- 6 458 745

## Description

### FIELD OF THE INVENTION:

The present invention relates to a process for preparing stable formulations of low melting agrochemicals. More particularly, the present invention relates to a granular formulation of low melting agrochemicals.

### BACKGROUND AND PRIOR ART:

Agrochemicals are used on a daily basis by farmers and farm workers. Handling and inhaling such chemicals can be injurious to health. For this reason, agrochemical formulators have developed various types of formulations which make handling of agrochemicals safer.

The use of agrochemical combinations is a widespread and documented practice in the agricultural community. These agrochemical combinations offer significant advantages over individual applications including improved and extended control, reduced application rates and costs, shorter contact times for improved results, less stringent use restrictions, improved selectivity, improved spectrum for fungi, insects, weeds etc. which are controlled, and reduced residue problems. However, identifying appropriate agrochemical application rates and ratio of the combinations is essential to achieve efficacious control. The selection of a particular formulation type is more cumbersome for an agrochemical combination as the stability and compatibility characteristics of both the agrochemicals need to be considered in selecting a formulation type.

Generally, the low melting agrochemicals are usually presented as liquid formulations. However, the prevalent agronomic practices lean more favorably towards use of a solid granular formulation over a liquid formulation for various reasons.

The granular formulations offer significant advantages over the liquid formulations such as ease of handling, ease of use, reduced worker exposure, reduced toxicity, reduced spillage and waste disposal, less field drift and therefore offer a convenient form for the delivery of the agrochemicals. These granules flow freely and cleanly from their containers and significantly reduce the dust hazards. In order to be effective, these granules are required to maintain their physical integrity until they are delivered to the carrier liquid, where they must disintegrate rapidly to fine particles that can remain suspended in the liquid phase.

Dispersants serve as suspending agents in wettable powders and perform the additional roles of binders. These dispersants increase the physical strength of the granules by improving the flow characteristics of the paste to reduce fissures and cracks, and by bridging between the particles to bind them together. Dispersants are known to be generally soluble in water and are counted as positive factors for rapid disintegration of the granules in water. However, the selection of the best dispersing agent for a particular pesticide or combinations thereof is critical and not certainly predictable.

An approach known to increase the attrition resistance of the granular formulation is by increasing the amount of the dispersing agent present in the formulation, as one of the primary roles of a dispersing agent in a wettable granular formulation is binding. However, increasing the amount of a dispersing agent is not always feasible because a formulation having a greater amount of a dispersing agent invariably requires a greater amount of time for dissolving. Thus, there is always a need to strike a balance between the need for attrition resistance and rapid dispersibility. There is always a need in the art for a wettable granular formulation of agrochemicals wherein the formulation displays superior attrition resistance, disperses rapidly in water and has good suspensibility in water once it is dispersed.

Another challenge to a formulator in preparing a granular formulation is the degree of chemical inertness of the granule towards the agrochemical. Moreover, it is not always reasonably predictable to select a carrier material that is simultaneously inert to two different agrochemicals present in the preferred combination. It has been observed that even if the carrier surface is mildly reactive to one of the agrochemical ingredients of the combination, it results into a breakdown of the other pesticide thereby reducing the efficacy of the formulation. Thus, the choice of an appropriate carrier for a granular formulation is often an undesired compromise based on an informed consideration of these characteristics of the known carrier materials.

There are three main processes known in the art for the preparation of a wettable granular formulation viz. spray drying, extrusion and fluid bed granulation. The formulations obtained by spray drying are free flowing, possess good dispersibility and exhibit no tendency to dusting. The spray drying process generally involves preparation of a slurry, spray drying of the prepared slurry and drying of the resulted granules. The slurry is often prepared by mixing the agrochemical ingredients with water, wetting and dispersing agents and other components including a dispersant, anti-foam, and stabilizers. This mixture is then homogenized by strong mechanical stirring or high shear mixer. The dispersion is then adjusted with water and milled in wet mill equipment in order to obtain a slurry. At this stage, the dynamic rheology of the slurry shows a pseudo-plastic behavior.

The slurry is thereafter sprayed through a bi-fluid nozzle or a pressure nozzle or an atomiser in large/small droplets. The process parameters during the spray drying process such as the inlet temperature, slurry flow rate, inlet air flow rate, type of nozzle etc. are critical and must be carefully selected to get the right residence time of the individual particles within the spray dryer. The dried spray granules are perfectly spherical. The granules are thereafter subjected to a discontinuous or continuous drying to get desired residual moisture (between 0.5% to 2%).

Problems in formulating low melting agrochemicals are known in the art, particularly, problems with preparing solid granular formulations of low melting agrochemicals. Most low melting agrochemicals are usually formulated as liquid formulations as they are difficult to stabilize and convert into solid formulations such as granules or dry flowable formulations. When formulated into such solid formulations they form suspensions with low suspensibility, stability and wettability, specifically in a dry flowable formulation. The most common method of formulating low melting agrochemicals is to absorb the agrochemical onto an inert material. US20090197765 (Gaytan et. al) discusses a solid formulation for low melting agrochemicals however; the application discloses the use of additional free flowing agents such as silicates. Such formulations, do not demonstrate higher levels of stability, or dispersibility or other performance characteristics. Moreover, the application discloses the use of such formulations for the herbicide fluroxypyr and its salts.

Another method known in the art is to mix surfactants with the agrochemical components, which in turn increase the dispersion properties of the agrochemicals. US5688743 (Essinger, Jr.**)** discusses a dry flowable composition for low melting solids and the use of nucleating agents such as surfactants which are admixed with the agrochemical and carriers so as to stabilize the agrochemical. However, such formulations do not demonstrate good dispersibility or suspensibility.

US5846903 (Lloyd**)** discloses a water dispersible granular formulation for low melting solids, which mixes surfactants with the agrochemicals and inert carriers. However, the drawback in such a formulation is that when the surfactant is blended into the mixture of agrochemical and carrier, the granules formed do not demonstrate good dispersibility or suspensibility.

The present invention aims to overcome the problems in the prior art, namely, formulating a dry flowable formulation for low melting agrochemicals, which can demonstrate excellent dispersibility, suspensibility and physical stability.

Surprisingly, it has been found that at least partially surface-coating granules of a low melting agrochemical with certain esters increases the physical stability of the agrochemical as well as demonstrates excellent dispersibility and suspensibility profile.

WO 2009/100101 discloses solid formulations comprising a low melting active compound. The solid formulations particularly can comprise pesticidal low melting compound, including herbicides, such as fluroxypyr and derivatives thereof. WO 2009/100101 further discloses methods of preparing the formulations and methods of plant treatment using the solid formulations.

WO 95/18531 discloses a formulation comprising: (a) at least one herbicide, (b) at least one porous solid carrier material, and (c) a nonionic surfactant, which formulation contains 30 to 60 % by weight, based on the total formulation, of at least one nonionic surfactant of the formula (I): R₁-(O-R₂-)nO-R₃, wherein R₁ is C₈-C₁₆ alkyl or C₈-C₁₆ alkenyl, R₂ is identical or different C₂-C₄ alkylene, R₃ is hydrogen or C₁-C₄ alkyl, and n is an integer from 5-10.

DE 102005051830 discloses solid formulations comprising: a) liquid or low melting polyalkoxylate and b) a high molecular weight sulfonate carrier, where (i) is at least 15% by weight of liquid or low melting polyalkoxylate based on the total weight of the solid formulation % by weight, (ii) the proportion of liquid or low melting polyalkoxylate based on the total weight of the higher molecular weight sulphonate is at least 30% by weight; (iii) the weight ratio of liquid or low melting polyalkoxylate to higher molecular weight sulfonate is at most 3:1. DE 102005051830 further discloses use of the solid formulations, in particular in the field of crop protection, and processes for producing such formulations.

WO 2012/009489 discloses stable herbicidal solid compositions containing built-in adjuvant which exhibit improved herbicidal efficacy when used to control weeds in flooded rice paddies or fields.

### SUMMARY OF THE INVENTION:

Therefore, in one aspect, the present invention provides a granular formulation according to claim 1.

In another aspect, the present invention provides a process for the preparation of a granular formulation according to claim 9.

In one embodiment, the present invention provides a process for the preparation of a granular formulation comprising a low melting agrochemical and at least a co-agrochemical, said process comprising:
(a) melting an agrochemically effective amount of a low melting agrochemical and sorbing molten low melting agrochemical on an absorbent to obtain particles of said low melting agrochemical;
(b) mixing an agrochemically effective amount of at least a co-agrochemical with at least an adjuvant to obtain particles of said co-agrochemical;
(c) optionally homogenously mixing said particles of low melting agrochemical and at least a co-agrochemical; and
(d) spray coating the particles with an ester prior to wet mixing, to give at least partially surface coating said particles of low melting agrochemical and the particles of co-agrochemical, wherein said ester is of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof.

In another aspect, the present invention provides a kit-of-parts according to claim 14.

### OBJECTS OF THE INVENTION:

It is an object of the present invention to provide a granular formulation of low melting agrochemicals.

It is another object of the present invention to provide a granular formulation that demonstrates excellent dispersibility and suspensibility.

It is another object of the present invention to provide a process for preparing a granular formulation comprising a low melting agrochemical.

It is yet another object of the present invention to provide an adjuvant system and process for surface coating low melting agrochemicals.

It is an object of the present invention to provide a granular formulation of low melting agrochemicals and at least a co-agrochemical.

It is another object of the present invention to provide a granular formulation of at least two agrochemicals that demonstrates excellent dispersibility and suspensibility.

It is another object of the present invention to provide a process for preparing a granular formulation comprising a low melting agrochemical and at least a co-agrochemical.

It is yet another object of the present invention to provide an adjuvant system and process for surface coating of a mixture of low melting agrochemical and at least a co-agrochemical.

### DETAILED DESCRIPTION OF THE INVENTION:

The present invention describes a dry flowable formulation for low melting agrochemicals and a process for preparing the same. The invention also describes an adjuvant system and process for surface-coating the granules with the adjuvant. The invention may also include another agrochemical(s). The other agrochemical may be a high melting agrochemicals stated as co-agrochemicals.

Accordingly, in one aspect, the present invention provides a granular formulation according to claim 1.

In another aspect, the present invention provides a process for the preparation of particles of a low melting agrochemical, according to claim 9.

The process thus involves melting an agrochemically effective amount of a low melting agrochemical and absorbing the melted agrochemical onto an absorbent so as to obtain particles of such a low melting agrochemical. Such particles may then be partially surface coated with an adjuvant system.

A low melting agrochemical is known in the art, and may be defined as a compound that has a melting point in the range of less than 100° C. Conversely, high melting agrochemicals may be defined as a compound that has a melting a point higher than 100°C.

The adjuvant system according to the instant invention that is used to at least partially surface coat the particles of agrochemicals comprises a phosphate ester of the compounds from the group comprising:
- (i): alkyl or aryl alkoxylate;
- (ii): alkoxylates of fatty alcohol;
- (iii): alkoxylate of fatty acids;
- (iv): block co-polymers of ethylene oxide and propylene oxide;
- (v): polyaryl substituted aliphatic or aromatic alkoxylate; and
- (vi): alkoxylated polyaryl substituted phenol; and derivatives and mixtures thereof.

In an embodiment, the ester is selected such that it has a degree of esterification from about 5 to about 95% and a pH in range of about 1 to 6.9.

As used herein, the term "aryl" shall include, for example, phenyl, tolyl, naphthyl, tetrahydronaphthyl, indanyl, indenyl, styryl, pyridyl, quinolinyl, and mixtures thereof.

The "alkoxylate" is preferably an ethoxylate though other alkoxylates are not excluded.

In yet another embodiment, suitable phosphated poly alkyl or aryl ethoxylate are styryl containing ethoxylated and phosphorylated phenols. More preferred are phenols having three styrene radicals and from about 16 to about 20 moles of ethylene oxide.

Without wishing to be bound by theory, the present inventors have surprisingly found that an alkoxylated surfactant selected from alkyl or aryl alkoxylate; alkoxylates of fatty alcohol; alkoxylate of fatty acids; block co-polymers of ethylene oxide and propylene oxide; polyaryl substituted aliphatic or aromatic alkoxylate; and alkoxylated polyaryl substituted phenol; and derivatives and mixtures thereof play an important role in the granular formulations comprising a low melting herbicide preferably but not limited to an oxyphenoxy acid ester herbicide optionally together with another agrochemical or co-agrochemical, preferably but not limited to a triazinone herbicide. It has been found that these classes of surfactants surprisingly enhance the physical performance such as dispersibility and suspensibility of the granular formulations envisaged herein. Still more surprisingly, it was found that when these classes of surfactants were mixed along with the remaining excipients and granulated to form particles comprising these surfactants within the particles and not as a surface-coating, the improved performance was absent.

It has now surprisingly been found that at least partially surface coating of said low melting agrochemical with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof with optionally at least one of the co-agrochemical exhibits a good dispersibility and suspensibility which is capable of providing the low melting agrochemical with or without high melting agrochemical along with quantitatively variable combination of adjuvants, in the form of solid formulations, preferably granules, for controlling the majority of weeds occurring preferably in useful plant cultivations, both in pre-emergence and in post-emergence, without significantly damaging the useful plants.

It is highly surprising that the combination of a modified styrene acrylic polymer with at least one of the following-mentioned surfactants selected from dialkyl naphthalene sulfonate formaldehyde condensate, dialkyl naphthalene sulfonate sodium salt, ethoxylated tristryl phenol, alkoxylate of fatty acids, sulphated poly aryl phenol ethoxylate, sodium sulphosuccinic acid di-isooctyl ester, sodium lauryl sulfate, polycarboxylate, sodium salt of naphthalene sulfonate condensate and others surfactants surpasses the additional effect to be expected in principle on the suspensibility for pests to be controlled, and thus extends the limits of activity of both active ingredients in particular in two different respects.

It was thus found that the performance of the granular formulations according to the instant invention was surprisingly enhanced when these surfactants were sprayed on the particles of low melting agrochemicals immediately prior to wet mixing for preparing a processable/granulable material. The enhanced performance characteristics were not found when these surfactants were added to the low melting agrochemical prior to the granulation. It is believed by the present inventors that spraying these surfactants on the particles of low melting agrochemicals immediately prior to the wet mixing results into at least a partial surface coating of the particles of low melting agrochemical and not within the particles. Without wishing to be bound by theory, it is believed that the ionically charged moieties of these classes of adjuvants/surfactants, when present within the particles, in close proximity to the low melting agrochemical and/or the co-agrochemical, chemically interacts to the ionically charged moieties of the agrochemicals and remains bound to the agrochemical molecules thereby failing to exhibit the performance enhancement otherwise seen due to the surface coating. It is believed that on surface coating of a low melting agrochemical, a portion of the surfactant releases and assists in dispersing the particles while a portion of the surfactant remains surface coated and stabilizes the low melting agrochemical. These classes of surfactants were indeed surprisingly selective in performing the dual role of dispersant as well as a stabilizer when present as at least a partial coating on the surface of the particles comprising a low melting agrochemical.

In another embodiment, the granular formulation of the present invention comprises a co-agrochemical, which may be a high melting agrochemical. Thus, in this embodiment, the present invention provides a granular formulation comprising particles of at least one low melting agrochemical admixed with particles of at least one co-agrochemical wherein said particles of low melting agrochemicals and optionally particles of at least one other agrochemical are at least partially surface coated with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof.

The low melting agrochemicals in an embodiment of the invention includes herbicides, insecticides and fungicides. Preferred classes include, amides, amidines, anilides, anilinopyrimidine, aryloxycarboxylic acids, aryloxyplenoxypropionates, benzilates, benzofurans, benzofuranylalkylsulfonates, benzothiazoles, benzoxazoles, benzoylpyrazoles, bipyridyliums, carbamates, carboxamides, chloroacetamides, chloroacetanilides, chlorotriazines, cyclodienes, cyclohexanediones, cyclohexane oximes, cyclopropylisoxazoles, dicarboximides, dinitroalilines, dinitrophenols, diphenyl ethers, dithiocarbamates, glycines, halogenated aliphatics, imidazoles, imidazolinones, isoazolidinones, neonicotinoids, nitroanilines, nitrophenylethers, N-phenylphthalimides, organoarsenicals, organichlorine, organophosphates, organophosphorus, oxadiazines, oxadiazolinones, oxazoles, oxyacetamides, phenoxyalkanoic acids, phenyl carbamates, phenoxyacetics, phenoxybutyrics, phenoxycarboxylic acids, phenoxypropionics, phenylamides, phenylpyrazoles, phenylpyridazines, phenylureas, phosphinic acids, phosphorodithioates, phthalic acids, pyrazoles, pyrethroids, pyridazines, pyridazinones, pyrimidines, pyridine carboxylic acids, pyrimidinamines, pyrimidinyloxybenzoic acids, pyrimidinylthiobenzoic acids, quaternary ammoniums, quinazolinones, quinoline carboxylic acids, strobilurins, sulfonamides, sulfonanilides, sulfonylaminocarbonyltriazolinones, sulfonylureas, tetranoic acid, thiaphanate, tetrazolinones, thiadiazoles, thiadiazolylureas, thiocarbamates, thiocarbonates, thioureas, triazines, triazoles, triazolinone, triazolones, triazinones, triazolopyrimidines, triketones, uracils, and ureas.

In another embodiment, the low melting agrochemical is selected from examples of agrochemicals which may be used in the process of the present invention will occur to those skilled in the art and include for example Alachlor, Alanycarb, Ametryn, Amitraz, Anilofos, Azamethiphos, Azinophos-ethyl, Azinophos-methyl, Beflubutamid, Benalaxyl, Benfluralin, Bensulide, Bensultap, Bifenox, Bifenthrin, Butralin, Butroxydim, Carboxin, Chlorpropham, Chlorpyrifos, Chlorpyrifos-methyl, Clodinafop-propargyl, Clofop-isobutyl, Cloquintocet-mexyl, Cyfluthrin, Beta-Cyfluthrin, Cyhalofop-butyl, Cypermethrin, Alpha-Cypermethrin, Beta-Cypermethrin, Cyprodinil, Diclofop-methyl, Dicofol, Difenoconazole, Diphenylamine, Dithiopyr, Ethofumesate, Etofenprox, Fenamiphos, Fenclorim, Fenfuram, Fenobucarb, Fenoxaprop, Fenoxaprop-ethyl, Fenoxaprop-P-ethyl, Fenoxycarb, Fenpropathrin, Fenrtazamide, Fenvalerate, Fluazinam, Fluazolate, Fluazifop-butyl, Fluchloralin, Flurenol, Fluroxypyr (-mepthyl), Flurprimidol, Haloxyfop-methyl, Heptachlor, Imazalil, Indoxacarb, Isoprocarb, Lactofen, Lambda-cyhalothrin, Linuron, MCPA-thioethyl, Mefenpyr-diethyl, Metazachlor, Methidathion, Methoxychlor, Monolinuron, Napropamide, Oxadiazon, Oxyfluorphen, Parathion-methyl, Pendimethalin, Permethrin, 2-Phenylphenol, Phosalone, Phosmet, Propachlor, Propanil, Propham, Pyrazophos, Quizalofop-P, Quizalopfop-methyl, Resmethrin, Pinosat, Temephos, Triadimefon, Triallate, Triazamate, Trifloxystrobin, Trifluralin.

In an embodiment, the low melting agrochemical is preferably selected from the class of oxyphenoxy acid ester herbicides selected from clodinafop-propargyl, fluazifop-butyl, fenoxaprop-ethyl, diclofop-methyl, quizalofop-methyl, haloxyfop-methyl and clofop-isobutyl. In yet another embodiment, the low melting agrochemical is clodinafop-propargyl.

In this embodiment, the preferred low melting compound is clodinafop-propargyl, which has the following structure:

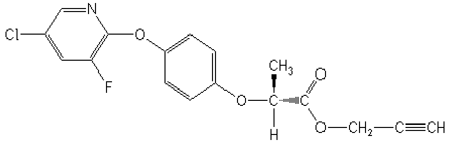

In one embodiment, the low melting agrochemical is clodinafop or its propargyl ester. Additionally, a safener may be used in combination with the low melting agrochemical. Such a safener may be selected from a list comprising of dymron, fenclorim, cumyluron, isoxadifen-ethyl, mefenpyr-diethyl, cloquintocet-mexyl, cyprosulfamide, dietholate, disulfoton, 1,8-naphthalic anhydride, fluxofenim, dichlormid, benoxacor, and flurazole.

In yet another embodiment, the safener is cloquintocet-mexyl.

In one embodiment the absorbing material may be precipitated silica or other silica based absorbing material, kaolin, soapstone, talc, starch, or a mixture thereof.

A co-agrochemical may be added to the formulation, such a co-agrochemical may have a high melting point. In an embodiment, the co-agrochemical may be selected from the list comprising of agrochemicals which may be used in the process of the present invention will occur to those skilled in the art and include for example Ametridione, Amibuzin, Ancymidol, Azaconazale, Azoxystrobin, Bendiocarb, Benoxacor, Bifenazate, Buprofezin, Butafenacil, Cafenstrole, Carbetamid, Chlordane, Chlorfenapyr, Chlozolinate, Cinidon-ethyl, Cyproconazole, 2,4-DB, DDT, Deltamethrin, Desmedipham, Dichlofluanid, Dichlorprop, Dichlorprop-P, Diethofencarb, Ethiozin, Ergocalciferol, Ethychlozate, Etoxazole, Fenarimol, Fenbuconazole, Fenchlorazole-ethyl, Fenclorim, Fenfuram, Fenpyroximate, Fentin, Fluazinam, Flumetralin, Fluthiacet-methyl, Flutolanil, Haloxyfop, Gamma HCH, Hexaconazole, Hexazinone, Hexythiazox, Imazamethabenz-methyl, Isoproturon, Isouron, Isomethiozin, Iprodione, Kinoprene, MCPA, Metamitron, Metribuzin, Metconazole, Methabenzthiazuron, Methiocarb, Metobenzuron, Neburon, Oxpoconazolefumerate, Pentoxazone, Picolinafen, Pindone, Polynactins, Prodiamine, Prometryn, Pyrazolynate, Pyrazoxyfen, Herbicide, Pyridaben, Pyriminobac-methyl, Quinoxyfen, Quizalofop, Sulfentrazone, Sulfur, Tebuconazole, Terbumeton, Terbutryn, Tralkoxydim, Trietazine, Trimethacarb, Vinclozolin In one embodiment, the co-agrochemical may be selected from the class of triazinone herbicides comprising ametridione, amibuzin, ethiozin, hexazinone, isoproturon, isomethiozin, metamitron and metribuzin.

In another embodiment, co-agrochemical is metribuzin, having the following structure:

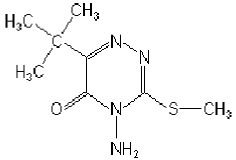

The present invention also provides a process for the preparation of particles of a low melting agrochemical. The process comprises:
(a) melting an agrochemically effective amount of a low melting agrochemical and sorbing the molten agrochemical on an absorbent to obtain particles of said low melting agrochemical; and
(b)at least partially surface coating said particles of low melting agrochemical with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof.

In an embodiment, the coated particles of low melting agrochemical are mixed with granulating/extruding aid to form an extrudable dough, which is thereafter extruded to form the partially surface coated granular particles of the present invention.

In another embodiment, the particles of low melting agrochemical may be co-formulated with the particles of at least a co-agrochemical. Thus, in this embodiment, the present invention provides a process for the preparation of a granular formulation comprising a low melting agrochemical and at leasta co-agrochemical, said process comprising:
(a) melting an agrochemically effective amount of a low melting agrochemical and sorbing molten agrochemical on an absorbent to obtain particles of said low melting agrochemical;
(b) mixing an agrochemically effective amount of at least a co-agrochemical with at least an adjuvant to obtain particles of said co-agrochemical;
(c) optionally homogenously mixing said particles of low melting agrochemical and at least a co-agrochemical; and
(d) at least partially surface coating said particles of low melting agrochemical and the particles of the co-agrochemical with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof.

In this aspect of the invention, the process may be used to formulate a low melting agrochemical along with a co-agrochemical which may be a high melting agrochemical. The process may be carried out by milling the high melting agrochemical to a desired size, mixing the high melting agrochemical with excipients, melting the low melting agrochemical and absorbing the said low melting agrochemical onto a suitable absorbing material, blending the low melting and high melting co-agrochemicals, treating the blend with an adjuvant system, mixing the adjuvant treated blend with a granulating/extruding aid and granulating/extruding or drying the same.

Thus, in one embodiment, the high melting co-agrochemical may be ground to a desired particle size using any one of the conventionally known milling equipment including air jet milling (AJM), air classifying mill (ACM), Turbo mill, Ultra fine mill (UFM), Hammer mill, Pin mill, Raymond mill, etc.

In an embodiment, the milled, the high melting co-agrochemical may be mixed with at least one excipient selected from dispersing agents, wetting agents, defoamers inert fillers, absorbers etc.

In an embodiment the dispersing agent may be selected from anionic surfactants such as modified styrene acrylic polymers.

In one embodiment, the formulation may contain ionic and nonionic dispersing agents to enable disintegration of granules in water with ease, such as modified styrene acrylic polymers, potassium polycarboxylates, salts of polystyrenesulphonic acids, salts of polyvinylsulphonic acids, salts of naphthalenesulphonic acid/formaldehyde condensates, salts of condensates of naphthalenesulphonic acid, phenolsulphonic acid and formaldehyde, and salts of lignosulphonic acid, polyethylene oxide/polypropylene oxide block copolymers, polyethylene glycol ethers of linear alcohols, reaction products of fatty acids with ethylene oxide and/or propylene oxide, polyvinyl alcohol, polyvinylpyrrolidone, copolymers of polyvinyl alcohol and polyvinylpyrrolidone and copolymers of (meth)acrylic acid and (meth)acrylic esters, alkyl ethoxylates and alkylarylethoxylates. In one embodiment, the dispersing agents include sodium naphthalene sulfonate-formaldehyde condensate, or a combination thereof. In another embodiment, the dispersing agent may include acrylic polymers or polycarboxylate potassium or a combination thereof.

In an embodiment the wetting agents may be selected from dialkyl naphthalene sulphonate sodium salt, sodium naphthalene sulfonate - formaldehyde condensate.

In another embodiment, the formulations of the present invention comprise at least one wetting agent selected from soaps; salts of aliphatic monoesters of sulphuric acid including but not limited to sodium lauryl sulphate, sulfoalkylamides and salts thereof including but not limited to N-methyl-N-oleoyltaurate Na salt; alkylarylsulfonates including but not limited to alkylbenzenesulfonates; alkylnaphthalenesulfonates and salts thereof and salts of ligninsulfonic acid.

In another embodiment, the wetting agent includes a blend comprising an alkali metal salt of alkylnaphthalenesulfonate or an alkali metal salt of dioctylsulphosuccinate or a combination thereof.

In an embodiment, the formulations of the present invention comprise at least one further surfactant selected from salts of polystyrenesulphonic acids; salts of polyvinylsulphonic acids; salts of naphthalenesulphonic acid/formaldehyde condensates; salts of condensates of naphthalenesulphonic acid, phenolsulphonic acid and formaldehyde; salts of lignosulphonic acid; polyethylene oxide/polypropylene oxide block copolymers; polyethylene glycol ethers of linear alcohols; reaction products of fatty acids with ethylene oxide and/or propylene oxide; polyvinyl alcohol; polyvinylpyrrolidone; copolymers of polyvinyl alcohol and polyvinylpyrrolidone; copolymers of (meth)acrylic acid and (meth)acrylic esters; and alkyl ethoxylates and alkylarylethoxylates.

In an embodiment the defoamers may be selected from silicone based defoamers.

In an embodiment, the formulations of the present invention may comprise at least one antifoaming agent which is usually employed for this purpose in agrochemical compositions.

In an embodiment, the preferred antifoaming agents are selected from silicone oil and magnesium stearate or a suitable combination thereof.

In an embodiment inert carrier may be selected form kaolin, titanium dioxide, bentonite, soapstone, talc, attapulgite, ceramic, montmorillonite, pumice, sepiolite, diatomaceous earth, sand, clay, dolomite, calcite, magnesium oxides, magnesium carbonates, sucrose, corn starch, citric acid and its salts, and mixtures thereof.

In an embodiment absorbers may be selected form silicates, precipitated silica, alumina, cellulose, activated carbon, graphite, silica gel, zeolites, bentonite, soapstone, talc etc, and mixture thereof.

In an embodiment, the low melting agrochemical and optionally a safener may be melted and absorbed onto a suitable absorbing material. The low melting agrochemical and safener may be absorbed onto an absorbent material by pouring, spraying or by using any suitable method and then allowed to rest for at least one hour after spraying. This is to allow proper absorption and even distribution of the agrochemical within the absorbing particles.

The process of absorbing may be carried out in a plough shear mixer, mixer grinder, kitchen mixer or any other conventional mixing equipment. In an embodiment the melted low melting agrochemical may be sprayed onto the absorbent material.

In an embodiment the co-agrochemical in an effective amount is mixed with at least an adjuvant to obtain particles of high melting agrochemical.

In an embodiment, homogenously mixing said particles of low melting agrochemical and at least a co-agrochemical of high melting group to have a blended mixture.

In an embodiment the blended mixture of the low and high melting agrochemicals may be at least partially coated with an adjuvant system.

In an embodiment the adjuvant system may be sprayed onto to the blended mixture in a suitable mixer like plough shear mixer, kitchen mixer, mixer grinder or so. The inventors of the present invention have found that adjuvant system when mixed with a low melting agrochemical and then granulated, gave very poor inversion and suspensibility profiles (as demonstrated in the examples below.) The inventors used various emulsifiers such as sodium lauryl sulphate, sodium ligosulphonates, and anionic surfactants that were mixed with the agrochemicals, specifically low melting agrochemicals which were present in concentrations of up to 25%. It has surprisingly been found that, when treated with an adjuvant system post mixing with dispersants, as well as other co-formulants, the granules demonstrated an excellent inversion and suspensibility profile.

In an embodiment, the coated blended powder may then be wet mixed with granulating/extruding aid and the subjected to known methods of granulation.

In an embodiment the solvents used in the process may include aqueous solvents or non aqueous solvents as granulating/extruding aid.

In an embodiment, the granules may be formed using any of the methods known in the art, such as pan granulation, high speed mixing agglomeration, extrusion granulation, fluid bed granulation, fluid bed spray granulation, spray drying. The preferred method is extrusion granulation. The extrusion granulation can be performed by using axial extruder, basket extruder, Fuji Paudal extruder, roller extruder, twin shaft extruder, low compact extruder, Eirich granulator, Oscillating granulator or any other suitable extruder to obtain the required quality of the granular product.

In an embodiment, the quantity of the agrochemicals, both the low melting and the high melting co-agrochemicals may be in the range of 1 to 25 % for low melting agrochemicals, whereas the high melting co-agrochemical may be in range of 5 to 50%. The term "agrochemically effective amount" as used herein shall be understood to define the above preferred amounts of the agrochemicals, although such amounts are not to be construed as limiting. It is within the reach of a person skilled in the art to determine the agrochemically effective amounts of one or more herbicide depending upon the crop, the infestation sought to be prevented, the environmental conditions etc.

The dry flowable granules formulated through the process mentioned above demonstrate excellent properties for both single agrochemicals such as low melting solids as well as combinations, wherein, a second high melting agrochemical may be a co-formulated.

The process is particularly useful for formulating low melting agrochemical in concentrations higher than 1 % by mass individually or in combination with the co-agrochemicals.

The process may be used to prepare dry flowables which are also known as water dispersible granules, as well as wettable granules. These granules can also be used either by direct broadcasting in the field (crop and or grass) or after dilution (with water or any other suitable diluents) to control the pest. The amount of formulation added to water to prepare a spray mixture may depend on the type of application. Usually a 0.1 to 10% by weight aqueous dispersion is prepared.

The invention therefore relates to a composition for controlling weeds and grasses in crops of cultivated plants, which composition contains a biologically effective amount of the novel formulation in water.

The dry flowable or water degradable granules of the present invention may be packaged as a kit of parts, wherein, the pre-formulated granules, which may be readily mixed with water, or in an embodiment, the kit of parts may contain such components such as a vial, bottle, can, pouch, bag or canister. In one embodiment, the kit of parts may contain pre-formulated granules of a low melting active and a high melting active packed separately, which may then be mixed in a vial or container or tank and sprayer before spraying. In one embodiment, the kit of parts may contain pre-formulated low melting active or high melting active, which may be readily mixed with water, and may include such components such as a vial, bottle, can, pouch, bag or canister.

Thus, in this aspect, the present invention provides a kit-of-parts for herbicidal treatment of plants, its habitat, a crop field, the soil or any material thereabout, said kit-of-parts comprising:
(a) a first herbicidal component, said first herbicidal component comprising particles of at least one low melting agrochemical, said particles being at least partially surface coated with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof;
(b) a second herbicidal component, said second herbicidal component comprising of at least a co-agrochemical, said particles being at least partially surface coated with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof; and
(c) an instruction manual comprising instructions for mixing the first and second herbicidal components in a predetermined ratio and treating the plants, its habitat, a crop field, the soil or any material thereabout with said admixture.

The first and second herbicidal components may be prepared by separately surface coating the particles of the low melting agrochemical and the particles of at least co-agrochemical with the adjuvant system of the present invention using a process described hereinabove.

The kit-of-parts according to an embodiment of the invention comprises an instruction manual. The instruction manual comprises instructions for an user of the kit to mix the first and second herbicidal components in a predetermined ratio. The preferred ratio is not particularly limiting and may be selected by skilled artisan according to the preferred dosage of the herbicides, the intensity of the infestations etc. The instruction manual also comprises instructions to disperse the admixture in a required quantity of water to prepare an aqueous suspension. The aqueous suspension is thereafter instructed to be sprayed to the plants, its habitat, a crop field, the soil or any material thereabout with said admixture.

The present examples demonstrate the excellent improvement in the suspensibility and dispersion profile of the granules obtained after treatment with an adjuvant system just prior to wet mixing or dough preparation, as well as decreased suspensibility and dispersion when adjuvants were mixed with the agrochemicals and formed into a homogenous powder that was then subjected to prepare wet extrudable dough for further subjection to granulation.

These and other advantages of the invention may become more apparent from the examples set forth herein below.

### Examples:

The present Example demonstrates the process for the preparation of particles of a low melting agrochemical and finally converting them into granules having good dispersibility and suspensibility. The compositions are as follows:

### Example 1

### Process A: -

Preparation of particles with low melting agrochemical and their respective granule:
a) Low melting agrochemical - Clodinafop propargyl
b) Safener - Cloquintocet mexyl
c) Dispersant- Dialkyl naphthalene sulfonate formaldehyde condensate or its sodium salt
d) Absorbent - Kaolin

### Other ingredients:

Adjuvant system - sulphated polyarylphenolethoxylate.
Inert filler - Lactose, ammonium sulphate

The above composition was prepared by following the present process as follows:
**Step a)** Clodinafop-propargyl technical and cloquintocet-mexyl safener were melted in a melting pot to obtain a homogenous mixture of these two.
**Step b)** Melted clodinafop propargyl and safener mixture was sprayed on to kaolin in a Plough-shear-mixer (PSM). The sprayed mix was then allowed to mix with a top-up quantity of precipitated silica to have a free flowing material free from lumps.
**Step d)** Homogenous mixture was then partially coated with adjuvant and/or stabilizer system prior to wet mixing.
**Step e)** Wet dough was prepared by mixing water (having defoamer) with the product of step (d), above, in required quantity to have the homogenous wet dough.
**Step f)** Dough from step (e), above, was then granulated using suitable granulator to obtain granules.
**Step g**) The granules, obtained from step (f) above, were then dried so as to ensure that the moisture content was below 5 %.

| Ingredients | Clodinafop Propargyl WP | | | | | |
|---|---|---|---|---|---|---|
| | 1% WG | 5% WG | 10% WG | 15% WG | 20% WG | 25% WG |
| | % by mass | | | | | |
| Clodinafop propargyl technical | 1.16 | 5.79 | 11.05 | 16.00 | 21.58 | 26.32 |
| Cloquintocet mexyl (safener) | 0.26 | 1.32 | 2.64 | 4.05 | 5.40 | 6.58 |
| Kaolin / Silica | 7.00 | 28.00 | 48.50 | 60.63 | 54.55 | 45.90 |
| Dialkyl naphthalene sulfonate salts (Supragil WP) | 1.50 | 5.00 | 8.50 | 9.00 | 10.50 | 10.50 |
| Lactose | 43.89 | 28.28 | 14.00 | 4.00 | 2.67 | 3.00 |
| Ammonium sulphate | 41.89 | 30.00 | 11.11 | - | - | - |
| Dimethyl polysiloxane (SAG 1572) | 0.20 | 0.21 | 0.20 | 0.32 | 0.30 | 0.20 |
| Sulphated polyarylphenol ethoxylate | 4.10 | 2.30 | 4.00 | 6.00 | 5.00 | 7.50 |
| Total | 100.0 | 100.0 | 100.0 | 100.0 | 100.0 | 100.0 |

| Process A | | | | | | |
|---|---|---|---|---|---|---|
| No. of inversions | 25 | 16 | 10 | 15 | 20 | 24 |
| Suspensibility (active) % by mass | 82.80 | 85.30 | 92.40 | 90.25 | 88.34 | 86.48 |
| Suspensibility (active) after 14 d-AHS | 80.37 | 84.65 | 92.00 | 92.51 | 85.94 | 88.22 |

**Process B:** To study the effect of adjuvant and/or stabilizer system when these adjuvant and/or stabilizer system was added in step (b) of Process A and rest steps were same.

| Ingredients | Clodinafop Propargyl WP | | | | | |
|---|---|---|---|---|---|---|
| | 1% WG | 5% WG | 10% WG | 15% WG | 20% WG | 25% WG |
| | % by mass | | | | | |
| Clodinafop propargyl technical | 1.16 | 5.79 | 11.05 | 16.00 | 21.58 | 26.32 |
| Kaolin / precipitated silica/ silica powder | 7.00 | 28.00 | 47.64 | 60.68 | 55.54 | 36.48 |
| Styrene acrylic polymer (Atlox Metasperse 550S) | 1.50 | 5.00 | 8.50 | 9.00 | 10.50 | 10.50 |
| Lactose | 43.89 | 27.78 | 14.00 | 4.00 | 2.67 | 3.00 |
| Ammonium sulphate | 41.89 | 30.00 | 11.11 | - | - | - |
| Sulphated polyarylphenol ethoxylate | 4.30 | 2.51 | 4.20 | 6.32 | 5.30 | 6.70 |
| Total | 100.0 | 100.0 | 100.0 | 100.0 | 100.0 | 100.0 |

| Process B | | | | | | |
|---|---|---|---|---|---|---|
| No. of inversions | 35 | 22 | 21 | 25 | 35 | 39 |
| Suspensibility (active) % by mass | 72.00 | 70.40 | 78.23 | 76.58 | 73.00 | 75.00 |
| Suspensibility (active) after 14 d-AHS | 54.00 | 45.00 | 59.00 | 64.00 | 61.08 | 60.00 |

**Process C**: To study the effect of adjuvant and/or stabilizer system when these adjuvant and/or stabilizer system was added in step (a) of Process A and rest steps were same.

| Ingredients | Clodinafop Propargyl WP | | | | | |
|---|---|---|---|---|---|---|
| | 1% WG | 5% WG | 10% WG | 15% WG | 20% WG | 25% WG |
| | % by mass | | | | | |
| Clodinafop propargyl technical | 1.16 | 5.79 | 11.05 | 16.00 | 21.58 | 26.32 |
| Cloquintocet mexyl (safener) | 0.26 | 1.32 | 2.64 | 4.05 | 5.40 | 6.58 |
| Kaolin/ Silica | 7.00 | 28.00 | 48.50 | 60.63 | 54.55 | 45.90 |
| Polycarboxylate derivative (Geropon SC-213) | 1.50 | 5.00 | 8.50 | 9.00 | 10.50 | 10.50 |
| Lactose | 41.89 | 25.28 | 10.00 | - | - | - |
| Ammonium sulphate | 43.89 | 32.50 | 15.11 | 4.00 | 2.67 | 3.00 |
| Sulphated polyarylphenol ethoxylate | 4.30 | 2.51 | 4.20 | 6.32 | 5.30 | 6.70 |
| Total | 100.0 | 100.0 | 100.0 | 100.0 | 100.0 | 100.0 |

| Process B | | | | | | |
|---|---|---|---|---|---|---|
| No. of inversions | 45 | 31 | 30 | 34 | >50 | >50 |
| Suspensibility (active) % by mass | 53.15 | 56.00 | 49.00 | 44.00 | 38.57 | 40.27 |
| Suspensibility (active) after 14 d-AHS | 40.20 | 44.11 | 38.75 | 36.46 | 30.21 | 29.70 |

### Example 2: Metribuzin 42% + Clodinafop-Proparqyl (12%) (DF)

### Preparation of particles with high melting agrochemical:

a) High melting agrochemical - Metribuzin (42%)
b) Dispersing agent - sodium naphthalene sulfonate-formaldehyde condensate (7%)
c) Wetting agent -sodium naphthalene sulfonate (4%)
d) Inert carrier- TiO₂ (Q.S)

### Preparation of particles with low melting agrochemical:

e) Low melting agrochemical - Clodinafop propargyl (12%)
f) Safener - Cloquintocet mexyl (3%)
g) Absorbent - Kaolin.

### Other ingredients:

Adjuvant added - sulphated polyaryl phenolethoxylate.

The above composition was prepared by following the present process as follows:
**Step a)** Metribuzin technical was ground to desired size using an Air-jet-mill.
**Step b)** Metribuzin technical was mixed with dispersing agent comprising sodium naphthalene sulfonate-formaldehyde condensate, Wetting agent comprising sodium naphthalene sulfonate and TiO₂ in a blender, this formed a premix of high melting co-agrochemical.
**Step c)** Clodinafop-propargyl technical and cloquintocet-mexyl safener were melted in a melting pot to obtain a homogenous mixture of the two.
**Step d)** Melted clodinafop-propargyl and safener mixture was sprayed on to precipitated silica or kaolin in a Plough-shear-mixer (PSM). The spray dried mix obtained formed a premix of low melting agrochemical.
**Step e)** The premix of both the high melting co-agrochemical and low melting agrochemical obtained in steps c and d were blended together in mixer like a plough-shear-mixer (PSM) with the additives to obtain a homogenous powder.
**Step f)** The homogenous powder obtained in step e was then treated with adjuvant system comprising polyaryl phenolethoxylateusing a plough-shear-mixer (PSM) to obtain a homogenous granulating mixture.
**Step g)** The homogenous granulating mixture of step f was mixed with water and a defoamer, such as silicone based deformers to form a homogenous wet dough.
**Step h)** The homogenous wet dough from step g, was then granulated using suitable granulator to obtain granules.
**Step i)** Granules, obtained from step h were then dried such that the moisture content was below 5 %.

### Example 3: Metribuzin 42% + Clodinafop-Proparqyl (12%) (DF)

### Preparation of particles with high melting agrochemical:

a) High melting agrochemical - Metribuzin (42%)
b) Dispersing agent - Modified styrene acrylic polymer (9%)

### Preparation of particles with low melting agrochemical:

a) Low melting agrochemical - Clodinafop propargyl (12%)
b) Safener - Cloquintocet mexyl (3%)
c) Absorbent - precipitated silica.
d) Wetting agent- sodium naphthalene sulfonate (4%)

### Other ingredients:

Adjuvants such as alkyl EO-PO block surfactant.

The above composition was prepared by following the present process as follows:
**Step a)** Metribuzin technical was ground to desired size using an Air-jet-mill.
**Step b)** Ground Metribuzin technical was mixed with dispersing agent comprising modified styrene acrylic polymer, which formed a premix of high melting co-agrochemicals.
**Step c)** Clodinafop-propargyl technical and cloquintocet-mexyl safener were melted in a melting pot to obtain a homogenous mixture of the two.
**Step d)** Melted clodinafop propargyl and safener mixture was sprayed on to precipitated silica mixed with wetting agent sodium naphthalene sulfonate in a plough-shear-mixer (PSM). The spray dried mix obtained formed a premix of low melting agrochemical.
**Step e)** The premix of both the high melting co-agrochemical and low melting agrochemical obtained in steps c and d were blended together in mixer like a plough-shear-mixer (PSM) with the additives to obtain a homogenous powder.
**Step f)** The homogenous powder was then treated with adjuvant comprising alkyl EO-PO block surfactant and their derivatives using a plough-shear-mixer (PSM) to obtain a homogenous granulating mixture.
**Step g)**The homogenous mixture obtained in step f, was then milled in a pin miller to obtain a homogenous free flowing powder.
**Step h)** The homogenous free flowing powder obtained in step g, was then mixed with a diluent such as water to obtain dough.
**Step i)** The dough obtained step h was then extruded and dried to obtain granules that have a moisture content was below 5 %.

### Example 4: Comparison of adjuvants.

| Ingredients | SET- A | SET - B | SET- C | | SET - D | |
|---|---|---|---|---|---|---|
| | 06/275 | 10/281 | 27B/308 | 32B/317 | 10BP/305 | 10CP/305 |
| Metribuzin technical | 44.30 | 46.67 | 44.50 | 44.50 | 43.30 | 43.30 |
| Precipitated Silica | 10.00 | - | 5.00 | 16.40 | - | - |
| Modified styrene acrylic polymer | 6.00 | 7.33 | 7.00 | 7.00 | 10.00 | 10.00 |
| Dialkyl naphthalene sulfonate formaldehyde condensate / sodium salt | 14.70 | 3.00 | - | - | 4.00 | 4.00 |
| Citric acid (CA) | - | - | 5.00 | 4.00 | 5.00 | 5.00 |
| Sodium bicarbonate (SBC) | - | - | 5.00 | 6.00 | 5.00 | 5.00 |
| Kaolin | 2.79 | 26.34 | 9.24 | - | 11.07 | 14.07 |
| Clodianfop propargyl | 12.95 | 13.33 | 13.00 | 13.68 | 13.00 | 13.00 |
| Cloquintocet mexyl | 3.26 | 3.33 | 3.26 | 3.42 | 3.26 | 3.26 |
| Ethoxylated tristyrylphenol | 6.00 | - | - | - | - | - |
| Sulphated polyarylphenolethoxylate | - | - | 5.00 | 4.00 | - | - |
| Sodium sulphosuccinic acid di-isooctyl ester | - | - | - | 1.00 | - | - |
| Extrusion aid | 8.00 (Water) | 10.00 (Water) | 2.00 (MEG) | 6.7 (MEG) | 3.00 (MEG) | 4.15 (water) |
| Inversion (in no.) a) (0 d) | 39 | 56 | 32 | 42 | 35 | 25 |
| b) after 14 days AHS | 40 | > 60 | 37 | >60 | >60 | >60 |
| Suspensibility (gravimetric) a) % (0d) Ambient | 82.00 | 21.61 | 88.00 | 81.50 | 45.62 | 24.58 |
| b) AHS (14 d) | 59.81 | 14.03 | 63.10 | 43.29 | 49.17 | 39.57 |

### Example 5:

| S. No. | Ingredients | SET E | | SET F | SET G | | |
|---|---|---|---|---|---|---|---|
| | | Sprayed adjuvant | Adjuvant mixed with agrochemical | Adjuvant sprayed on powder | No Adjuvant sprayed or added to the formulation. | | |
| | | (20+6) 02/271 | (42+12) 06/275 | (30+8) 03/272 | (42+12) 09/280 | (42+12) 10/281 | (42+12) 11/282 |
| 1 | Metribuzin technical | 21.0 | 44.3 | 32.0 | 43.59 | 46.68 | 44.30 |
| 2 | Precipitated Silica | - | 10.0 | 5.0 | 4.65 | - | 4.00 |
| 3 | Dialkyl naphthalene sulfonate sodium salt | 14.0 | 16.0 | 14.0 | 9.94 | 10.33 | 10.00 |
| 4 | Kaolin | 30.0 | 5.49 | 32.0 | 22.60 | 26.33 | 10.22 |
| 5 | Clodinafop propargyl | 7.0 | 12.95 | 8.3 | 12.82 | 13.33 | 13.00 |
| 6 | Cloquintocet mexyl | 1.5 | 3.26 | 2.2 | 3.19 | 3.33 | 3.26 |
| 7 | Ethoxylated tristyrylphenol | - | 6.0 | 6.5 | - | - | - |
| 8 | Castor oil ethoxylate | 8.5 | 2.0 | - | - | - | - |
| 9 | Ammonium sulphate | 18.0 | - | - | - | - | - |
| 10 | sodium alkyl naphthalene sulfonate blend | - | - | - | 3.21 | - | |
| 11 | sodium salt of naphthalene sulfonate condensate | - | - | - | - | - | 15.22 |
| | Remarks | | | | AJM -No melting | AJM - No melting | AJM - No melting |
| A | Extrusion aid | Water | 8.00 (Water) | Water | 6.8 Water | 9.5 Water | 9.0 Water |
| B | Inversion (no.) (0 d) | 28 | 39 | 24 | >30 | 56 | 43 |
| | AHS | N/A | 40 | N/A | N/A | N/A | N/A |
| C | Suspensibility (gravimetric) % (0d) Ambient | 82.61 (0d) | 62.0 (0d) | 95.50/97.42 | 26.42 | 21.61 | 46.76 |
| | AHS (14 d) | N/A | 59.81 | N/A | N/A | N/A | N/A |

Sets E, F and G clearly demonstrate the effect of the adjuvant system on the suspensibility. Set E was divided into two parts; one part was formulated by spraying the adjuvant system onto the homogenous powder, and the other half was formulated by mixing the adjuvant system mixed with agrochemical whilst blending. The results demonstrate that sprayed adjuvant or a coating of the adjuvant onto the homogenous powder, resulted in superior suspensibility.

Set F also demonstrated that spraying adjuvant onto the homogenous powder gave granules with excellent suspensibility as compared to those formulations prepared in Set G (No adjuvant) and Set E (part one).

## Claims

1. A granular formulation comprising particles of at least one low melting agrochemical, selected from the group consisting of oxyphenoxy acid ester compounds selected from the group consisting of clodinafop-propargyl, fluazifopbutyl, fenoxaprop-ethyl, diclofop-methyl, quizalofop-methyl, haloxyfop-methyl and clofop-isobutyl, admixed with particles of at least one co-agrochemical, selected from the group consisting of a triazinone herbicide selected from ametridione, amibuzin, ethiozin, hexazinone, isomethiozin, metamitron and metribuzin in an amount of 1% to 25%, wherein said particles of low melting agrochemicals and optionally particles of at least one co-agrochemical in an amount of about 5% to 50% are at least partially surface coated by spraying with an ester prior to wet mixing, wherein said ester is of a compound selected from the group comprising (a) an alkyl or aryl alkoxylate, (b) alkoxylates of fatty alcohol, (c) alkoxylates of fatty acids, (d) block co-polymers of alkyls or ethylene oxides and propylene oxides, (e) polyaryl substituted aliphatic or aromatic alkoxylate, (f) alkoxylated polyaryl substituted phenol and their derivative and/or mixtures thereof.

2. The formulation as claimed in claim 1 further comprising at least one safener selected from the group consisting of dymron, fenclorim, cumyluron, isoxadifenethyl, mefenpyr-diethyl, cloquintocet-mexyl, cyprosulfamide, dietholate, disulfoton, 1,8-naphthalic anhydride, fluxofenim, dichlormid, benoxacor, and flurazole.

3. The formulation as claimed in any one of the preceding claims, wherein the particles of low melting agrochemical or the particles of at least a co-agrochemical comprise at least one excipient selected from dispersing agents, wetting agents, defoamers, inert fillers and absorbers.

4. The formulation as claimed in claim 3, wherein the dispersing agent is modified styrene acrylic polymer or polycarboxylate potassium.

5. The formulation as claimed in claim 3 or claim 4, wherein the wetting agent is selected from dialkyl naphthalene sulphonate formaldehyde condensate, dialkyl naphthalene sulphonate sodium salt and sodium naphthalene sulfonate - formaldehyde condensate.

6. The formulation as claimed in claims 3-5, wherein the defoamer is a silicon based defoamer.

7. The formulation as claimed in any one of the preceding claims, wherein the particles of low melting agrochemical are absorbed on an absorbent selected from silicate, precipitated silica, kaolin, bentonite and a mixture thereof.

8. The granular formulation as claimed in claim 1, wherein the particles of low melting agrochemical are at least partially surface coated with a phosphate of polystyryl phenol ethoxylate.

9. A process for the preparation of a granular formulation comprising a low melting agrochemical, selected from the group consisting of oxyphenoxy acid ester compounds selected from the group consisting of clodinafop-propargyl, fluazifopbutyl, fenoxaprop-ethyl, diclofop-methyl, quizalofop-methyl, haloxyfop-methyl and clofop-isobutyl, said process comprising: (a) melting an agrochemically effective amount of a low melting agrochemical and sorbing the molten agrochemical on an absorbent to obtain particles of said low melting agrochemical; and (b) spray coating said particles with an ester prior to wet mixing, to give at least partially surface coated particles of low melting agrochemical, wherein said ester is of a compound selected from the group comprising (a) an alkyl or aryl alkoxylate, (b) alkoxylates of fatty alcohol, (c) alkoxylates of fatty acids, (d) block co-polymers of alkyl or ethylene oxides or propylene oxides, (e) polyaryl substituted aliphatic or aromatic alkoxylate, (f) alkoxylated polyaryl substituted phenol and their derivative and/or mixtures thereof.

10. The process of claim 9, wherein the granular formulation further comprises at least a co-agrochemical, selected from the group consisting of a triazinone herbicide selected from ametridione, amibuzin, ethiozin, hexazinone, isomethiozin, metamitron and metribuzin, and the process further comprises (b) mixing an agrochemically effective amount of at least a co-agrochemical with at least an adjuvant to obtain particles of said co-agrochemical; (c) optionally homogenously mixing said particles of low melting agrochemical and at least a co-agrochemical; and (d) spray coating the particles of a co-agrochemical with an ester prior to wet mixing, to give at least partially surface coated particles of a co-agrochemical, wherein said ester is of a compound selected from the group comprising (a) an alkyl or aryl alkoxylate, (b) alkoxylates of fatty alcohol, (c) alkoxylates of fatty acids, (d) block co-polymers of ethylene oxides and propylene oxides, (e) polyaryl substituted aliphatic or aromatic alkoxylate, (f) alkoxylated polyaryl substituted phenol and their derivative and/or mixtures thereof.

11. The process as claimed in claim 9 or claim 10, wherein the process comprises admixing the partially surface coated particles of low melting agrochemicals and optionally particles of at least co-agrochemical with an aqueous solvent to obtain a wet mix.

12. The process as claimed in claim 11, wherein the process comprises (a) granulating /extruding the obtained wet mix and drying the granulated/extruded wet particles so obtained.

13. The process as claimed in claim 9 or any one of the claims 10-12, wherein sorbing the low melting agrochemical on an absorbent comprises spraying the molten low melting agrochemical on the absorbing material in a plough shear mixer.

14. A kit-of-parts for herbicidal treatment of plants, its habitat, a crop field, the soil or any material thereabout, said kit-of-parts comprising: (a) a first herbicidal component, said first herbicidal component comprising particles of at least one low melting agrochemical, selected from the group consisting of oxyphenoxy acid ester compounds selected from the group consisting of clodinafop-propargyl, fluazifop-butyl, fenoxaprop-ethyl, diclofop methyl, quizalofop-methyl, haloxyfop-methyl and clofop-isobutyl, said particles being spray coated prior to wet mixing, to give particles at least partially surface coated with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof; (b) a second herbicidal component, said second herbicidal component comprising of at least a co-agrochemical, selected from the group consisting of a triazinone herbicide selected from ametridione, amibuzin, ethiozin, hexazinone, isomethiozin, metamitron and metribuzin, said particles being spray coated prior to wet mixing, to give particles at least partially surface coated with an ester of a compound selected from the group comprising (a) an alkyl or aryl alkoxylate, (b) alkoxylates of fatty alcohol, (c) alkoxylates of fatty acids, (d) block co-polymers of alkyls, or ethylene oxides or propylene oxides, (e) polyaryl substituted aliphatic or aromatic alkoxylate, (f) alkoxylated polyaryl substituted phenol and their derivative and/or mixtures thereof; and (c) an instruction manual comprising instructions for mixing the first and second herbicidal components in a predetermined ratio and treating the plants, its habitat, a crop field, the soil or any material thereabout with said admixture.

## Patentansprüche

1. Granuläre Formulierung, umfassend Partikel von mindestens einer gering schmelzenden Agrochemikalie, die ausgewählt ist aus der Gruppe bestehend aus Oxyphenoxysäureesterverbindungen, die aus der Gruppe bestehend aus Clodinafoppropargyl, Fluazifopbutyl, Fenoxapropethyl, Diclofopmethyl, Chizalofopmethyl, Haloxyfopmethyl und Clofopisobutyl ausgewählt sind, gemischt mit Partikeln von mindestens einer Coagrochemikalie, die aus der Gruppe bestehend aus einem Triazinonherbizid ausgewählt ist, das aus Ametridion, Amibuzin, Ethiozin, Hexazinon, Isomethiozin, Metamitron und Metribuzin in einer Menge von 1 % bis 25 % ausgewählt ist, wobei die Partikel aus gering schmelzenden Agrochemikalien und gegebenenfalls Partikel aus mindestens einer Coagrochemikalie in einer Menge von etwa 5 % bis 50 % zumindest teilweise durch Bespritzen mit einem Ester vor dem feuchten Mischen oberflächenbeschichtet werden, wobei der Ester von einer Verbindung ist, die aus der Gruppe ausgewählt ist, die (a) ein Alkyl oder Arylalkoxylat, (b) Alkoxylate von Fettalkohol, (c) Alkoxylate von Fettsäuren, (d) Blockcopolymere von Alkylen oder Ethylenoxide und Propylenoxide, (e) mit Polyaryl substituiertes aliphatisches oder aromatisches Alkoxylat, (f) alkoxyliertes, mit Polyaryl substituiertes Phenol und ihr Derivat und/oder Gemische davon umfasst.

2. Formulierung nach **Anspruch** 1, ferner umfassend mindestens einen Safener, der aus der Gruppe bestehend aus Dymron, Fenclorim, Cumyluron, Isoxadifenethyl, Mefenpyrdiethyl, Clochintocetmexyl, Cyprosulfamid, Dietholat, Disulfoton, 1,8-Naphthalanhydrid, Fluxofenim, Dichlormid, Benoxacor und Flurazol ausgewählt ist.

3. Formulierung nach einem der vorhergehenden Ansprüche, wobei die Partikel von gering schmelzender Agrochemikalie oder die Partikel von mindestens einer Coagrochemikalie mindestens einen Hilfsstoff umfasst, der aus Dispergiermitteln, Benutzungsmitteln, Entschäumern, inerten Füllstoffen und Absorbiermitteln ausgewählt ist.

4. Formulierung nach Anspruch 3, wobei das Dispergiermittel modifiziertes Styrol-Acryl-Polymer oder Polycarboxylatkalium ist.

5. Formulierung nach Anspruch 3 oder Anspruch 4, wobei das Benetzungsmittel aus Dialkylnaphthalinsulfonatformaldehydkondensat, Dialkylnaphthalinsulfonatnatriumsalz und Natriumnaphthalinsulfonatformaldehydkondensat ausgewählt ist.

6. Formulierung nach Anspruch 3-5, wobei der Entschäumer ein Entschäumer auf Siliciumbasis ist.

7. Formulierung nach einem der vorhergehenden Ansprüche, wobei die Partikel von gering schmelzender Agrochemikalie auf einem Absorptionsmittel absorbiert werden, das aus Silicat, gefälltem Siliciumdioxid, Kaolin, Bentonit und einem Gemisch davon ausgewählt ist.

8. Granuläre Formulierung nach Anspruch 1, wobei die Partikel von gering schmelzender Agrochemikalie zumindest teilweise mit einem Phosphat von Polystyrylphenolethoxylat oberflächenbeschichtet sind.

9. Verfahren zur Herstellung einer granulären Formulierung, die eine gering schmelzende Agrochemikalie umfasst, die aus der Gruppe bestehend aus Oxyphenoxysäureesterverbindungen ausgewählt ist, die aus der Gruppe bestehend aus Clodinafoppropargyl, Fluazifopbutyl, Fenoxapropethyl, Diclofopmethyl, Chizalofopmethyl, Haloxyfopmethyl und Clofopisobutyl ausgewählt sind, wobei das Verfahren Folgendes umfasst: (a) Schmelzen einer agrochemisch wirksamen Menge einer gering schmelzenden Agrochemikalie und Sorbieren der geschmolzenen Agrochemikalie auf einem Absorptionsmittel unter Erhalt von Partikeln der gering schmelzenden Agrochemikalie; und (b) Spritzbeschichten der Partikel mit einem Ester vor dem feuchten Mischen unter Erhalt zumindest teilweise oberflächenbeschichteter Partikel aus gering schmelzender Agrochemikalie, wobei der Ester von einer Verbindung ist, die aus der Gruppe ausgewählt ist, die (a) ein Alkyl oder Arylalkoxylat, (b) Alkoxylate von Fettalkohol, (c) Alkoxylate von Fettsäuren, (d) Blockcopolymere von Alkyl oder Ethylenoxiden oder Propylenoxiden, (e) mit Polyaryl substituiertes aliphatisches oder aromatisches Alkoxylat, (f) alkoxyliertes, mit Polyaryl substituiertes Phenol und ihr Derivat und/oder Gemische davon umfasst.

10. Verfahren nach Anspruch 9, wobei die granuläre Formulierung ferner mindestens eine Coagrochemikalie umfasst, die aus der Gruppe bestehend aus einem Triazinonherbizid ausgewählt ist, das aus Ametridion, Amibuzin, Ethiozin, Hexazinon, Isomethiozin, Metamitron und Metribuzin ausgewählt ist, und das Verfahren ferner Folgendes umfasst: (b) Mischen einer agrochemisch wirksamen Menge von mindestens einer Coagrochemikalie mit mindestens einem Adjuvans unter Erhalt von Partikeln der Coagrochemikalie; (c) gegebenenfalls homogenes Mischen der Partikel aus gering schmelzender Agrochemikalie und mindestens einer Coagrochemikalie; und (d) Spritzbeschichten der Partikel aus Coagrochemikalie mit einem Ester vor dem feuchten Mischen unter Erhalt zumindest teilweise oberflächenbeschichteter Partikel einer Coagrochemikalie, wobei der Ester von einer Verbindung ist, die aus der Gruppe ausgewählt ist, die (a) ein Alkyl oder Arylalkoxylat, (b) Alkoxylate von Fettalkohol, (c) Alkoxylate von Fettsäuren, (d) Blockcopolymere von Ethylenoxide und Propylenoxide, (e) mit Polyaryl substituiertes aliphatisches oder aromatisches Alkoxylat, (f) alkoxyliertes, mit Polyaryl substituiertes Phenol und ihr Derivat und/oder Gemische davon umfasst.

11. Verfahren nach Anspruch 9 oder Anspruch 10, wobei das Verfahren ein Mischen der teilweise oberflächenbeschichteten Partikel aus gering schmelzenden Agrochemikalien und gegebenenfalls Partikeln von zumindest Coagrochemikalie mit einem wässrigen Lösungsmittel unter Erhalt einer feuchten Mischung umfasst.

12. Verfahren nach Anspruch 11, wobei das Verfahren ein (a) Granulieren/Extrudieren des erhaltenen feuchten Gemischs und ein Trocknen der auf diese Weise erhaltenen granulierten/extrudierten feuchten Partikel umfasst.

13. Verfahren nach Anspruch 9 oder einem beliebigen der Ansprüche 10-12, wobei das Sorbieren der gering schmelzenden Agrochemikalie auf einem Absorptionsmittel ein Aufspritzen der geschmolzenen gering schmelzenden Agrochemikalie auf das Absorptionsmaterial in einem Flügelschermischer umfasst.

14. Zubehörkit für die Herbizidbehandlung von Pflanzen, ihrem Habitat, einem Getreidefeld, der Erde oder irgendeinem Material dafür, wobei das Zubehörkit Folgendes umfasst: (a) eine erste Herbizidkomponente, wobei die erste Herbizidkomponente Partikel aus mindestens einer gering schmelzenden Agrochemikalie umfasst, die aus der Gruppe bestehend aus Oxyphenoxysäureesterverbindungen ausgewählt ist, die aus der Gruppe bestehend aus Clodinafoppropargyl, Fluazifopbutyl, Fenoxapropethyl, Diclofopmethyl, Chizalofopmethyl, Haloxyfopmethyl und Clofopisobutyl ausgewählt sind, wobei die Partikel vor dem feuchten Mischen unter Erhalt von Partikeln spritzbeschichtet werden, die zumindest teilweise mit einem Ester einer Verbindung oberflächenbeschichtet sind, die aus der Gruppe ausgewählt ist, die (a) ein Alkyl oder Arylalkoxylat, (b) Alkoxylate von Fettalkohol, (c) Alkoxylate von Fettsäuren, (d) Blockcopolymere von Alkylen oder Ethylenoxide und Propylenoxide, (e) mit Polyaryl substituiertes aliphatisches oder aromatisches Alkoxylat, (f) alkoxyliertes, mit Polyaryl substituiertes Phenol und ihr Derivat und/oder Gemische davon umfasst; (b) eine zweite Herbizidkomponente, wobei die zweite Herbizidkomponente zumindest eine gering schmelzende Coagrochemikalie umfasst, die aus der Gruppe bestehend aus Triazinonherbizid ausgewählt ist, das aus Ametridion, Amibuzin, Ethiozin, Hexazinon, Isomethiozin, Metamitron und Metribuzin ausgewählt ist, wobei die Partikel vor dem feuchten Mischen unter Erhalt von Partikeln spritzbeschichtet werden, die zumindest teilweise mit einem Ester einer Verbindung oberflächenbeschichtet sind, die aus der Gruppe ausgewählt ist, die (a) ein Alkyl oder Arylalkoxylat, (b) Alkoxylate von Fettalkohol, (c) Alkoxylate von Fettsäuren, (d) Blockcopolymere von Alkylen oder Ethylenoxide und Propylenoxide, (e) mit Polyaryl substituierten aliphatischen oder aromatischen Alkoxylat, (f) alkoxyliertes, mit Polyaryl substituiertem Phenol und ihr Derivat und/oder Gemische davon umfasst; und (c) eine Gebrauchsanweisung, die Anweisungen zum Mischen der ersten und der zweiten Herbizidkomponente in einem vorab festgelegten Verhältnis und zum Behandeln der Pflanzen, ihres Habitats, eines Getreidefelds, der Erde oder irgendeines Material dafür mit dem Gemisch umfasst.

## Revendications

1. Formulation granulaire comprenant des particules d'au moins un agent agrochimique à point de fusion bas, sélectionné dans le groupe constitué de composés d'ester d'acide oxyphénoxylique sélectionnés dans le groupe constitué du clodinafop-propargyle, du fluazifopbutyle, du fénoxaprop-éthyle, du diclofop-méthyle, du quizalofop-méthyle, de l'haloxyfop-méthyle et du clofop-isobutyle, mélangés avec des particules d'au moins un co-agent agrochimique, sélectionné dans le groupe constitué d'un herbicide à base de triazinone sélectionné parmi l'amétridione, l'amibuzin, l'éthiozine, l'hexazinone, l'isométhiozine, le métamitron et la métribuzine en une quantité de 1 % à 25 %, dans laquelle lesdites particules d'agents agrochimiques à point de fusion bas et éventuellement des particules d'au moins un co-agent agrochimique en une quantité d'environ 5 % à 50 % sont au moins partiellement revêtues en surface par pulvérisation avec un ester avant un mélange à l'état humide, dans laquelle ledit ester est un composé sélectionné dans le groupe comprenant (a) un alcoxylate d'alkyle ou d'aryle, (b) des alcoxylates d'alcool gras, (c) des alcoxylates d'acides gras, (d) des copolymères séquencés d'alkyles, ou des oxydes d'éthylène et des oxydes de propylène, (e) un alcoxylate aliphatique ou aromatique substitué par un polyaryle, (f) un phénol substitué par un polyaryle alcoxylé et leurs dérivés et/ou des mélanges de ceux-ci.

2. Formulation selon la revendication 1, comprenant en outre au moins un phytoprotecteur sélectionné parmi le groupe constitué du dymron, du fenclorim, du cumyluron, de l'isoxadifen-éthyl, le méfenpyr-diéthyl, le cloquintocet-mexyl, le cyprosulfamide, le diétholate, le disulfoton, l'anhydride 1,8-naphtalique, la fluxofénime, le dichlormide, le bénoxacor et le flurazole.

3. Formulation selon l'une quelconque des revendications précédentes, dans laquelle les particules d'agent agrochimique à point de fusion bas ou les particules d'au moins un coagent agrochimique comprennent au moins un excipient sélectionné parmi des agents dispersants, des agents mouillants, des agents antimousses, des charges inertes et des absorbeurs.

4. Formulation selon la revendication 3, dans laquelle l'agent dispersant est un polymère styrène-acrylique modifié ou du polycarboxylate de potassium.

5. Formulation selon la revendication 3 ou la revendication 4, dans laquelle l'agent mouillant est sélectionné parmi un condensat de dialkyl-naphtalène-sulfonate-formaldéhyde, un sel sodique de dialkyl-naphtalène-sulfonate et un condensat de naphtalène-sulfonate de sodium - formaldéhyde.

6. Formulation selon l'une des revendications 3 à 5, dans laquelle l'agent antimousse est un agent antimousse à base de silicium.

7. Formulation selon l'une quelconque des revendications précédentes, dans laquelle les particules d'agent agrochimique à point de fusion bas sont absorbées sur un absorbant sélectionné parmi le silicate, la silice précipitée, le kaolin, la bentonite et un mélange de ceux-ci.

8. Formulation granulaire selon la revendication 1, dans laquelle les particules d'agent agrochimique à point de fusion bas sont au moins partiellement revêtues en surface avec un phosphate d'éthoxylate de polystyryl-phénol.

9. Procédé de préparation d'une formulation granulaire comprenant un agent agrochimique à point de fusion bas, sélectionné dans le groupe constitué de composés d'ester d'acide oxyphénoxylique sélectionnés dans le groupe constitué du clodinafop-propargyle, du fluazifopbutyle, du fénoxaprop-éthyle, du diclofop-méthyle, du quizalofop-méthyle, de l'haloxyfop-méthyle et du clofop-isobutyle, ledit procédé comprenant : (a) la fusion d'une quantité efficace sur le plan agrochimique d'un agent agrochimique à point de fusion bas et l'absorption de l'agent agrochimique fondu sur un absorbant pour obtenir des particules dudit agent agrochimique à point de fusion bas ; et (b) le revêtement par pulvérisation desdites particules avec un ester avant un mélange à l'état humide, pour donner des particules au moins partiellement revêtues en surface d'un agent agrochimique à point de fusion bas, dans laquelle ledit ester est un composé sélectionné dans le groupe comprenant (a) un alcoxylate d'alkyle ou d'aryle, (b) des alcoxylates d'alcool gras, (c) des alcoxylates d'acides gras, (d) des copolymères séquencés d'alkyles, ou des oxydes d'éthylène ou des oxydes de propylène, (e) un alcoxylate aliphatique ou aromatique substitué par un polyaryle, (f) un phénol substitué par un polyaryle alcoxylé et leurs dérivés et/ou des mélanges de ceux-ci.

10. Procédé selon la revendication 9, dans lequel la formulation granulaire comprend en outre au moins un coagent agrochimique, sélectionné dans le groupe constitué d'un herbicide à base de triazone sélectionné parmi l'amétridione, l'amibuzin, l'éthiozine, l'hexazinone, l'isométhiozine, le métamitron et la métribuzine, et le procédé comprend en outre (b) le mélange d'une quantité efficace sur le plan agrochimique d'au moins un coagent agrochimique avec au moins un adjuvant pour obtenir des particules dudit coagent agrochimique ; (c) éventuellement le mélange homogène desdites particules d'un agent agrochimique à point de fusion bas et d'au moins un coagent agrochimique ; et (d) le revêtement par pulvérisation des particules d'un coagent agrochimique avec un ester avant un mélange à l'état humide, pour donner des particules au moins partiellement revêtues en surface d'un coagent agrochimique, dans lequel ledit ester est un composé sélectionné dans le groupe comprenant (a) un alcoxylate d'alkyle ou d'aryle, (b) des alcoxylates d'alcool gras, (c) des alcoxylates d'acides gras, (d) des copolymères séquencés d'oxydes d'éthylène et des oxydes de propylène, (e) un alcoxylate aliphatique ou aromatique substitué par un polyaryle, (f) un phénol substitué par un polyaryle alcoxylé et leurs dérivés et/ou des mélanges de ceux-ci.

11. Procédé selon la revendication 9 ou la revendication 10, dans lequel le procédé comprend le mélange des particules partiellement revêtues en surface d'agents agrochimiques à point de fusion bas et éventuellement des particules d'au moins un co-agent agrochimique avec un solvant aqueux pour obtenir un mélange à l'état humide.

12. Procédé selon la revendication 11, dans lequel le procédé comprend (a) la granulation / l'extrusion du mélange à l'état humide obtenu et le séchage des particules humides granulées/extrudées ainsi obtenues.

13. Procédé selon la revendication 9 et l'une quelconque des revendications 10 à 12, dans lequel l'absorption de l'agent agrochimique à point de fusion bas sur un absorbant comprend la pulvérisation de l'agent agrochimique à point de fusion bas fondu sur le matériau absorbant dans un mélangeur à cisailles de charrue.

14. Kit de composants pour un traitement herbicide de plantes, leur habitat, un champ de culture, le sol ou tout matériau autour de ceux-ci, ledit kit de composants comprenant : (a) un premier composant herbicide, ledit composant herbicide comprenant des particules d'au moins un agent agrochimique à point de fusion bas, sélectionné dans le groupe constitué de composés d'ester d'acide oxyphénoxylique sélectionnés dans le groupe constitué du clodinafop-propargyle, du fluazifopbutyle, du fénoxaprop-éthyle, du diclofop-méthyle, du quizalofop-méthyle, de l'haloxyfop-méthyle et du clofop-isobutyle, lesdites particules étant revêtues par pulvérisation avant un mélange à l'état humide, pour donner des particules au moins partiellement revêtues en surface avec un ester d'un composé sélectionné dans le groupe comprenant (a) un alcoxylate d'alkyle ou d'aryle, (b) des alcoxylates d'alcool gras, (c) des alcoxylates d'acides gras, (d) des copolymères séquencés d'alkyles, des oxydes d'éthylène ou des oxydes de propylène, (e) un alcoxylate aliphatique ou aromatique substitué par un polyaryle, (f) un phénol substitué par un polyaryle alcoxylé et leurs dérivés et/ou des mélanges de ceux-ci ; (b) un second composant herbicide, ledit second composant herbicide comprenant au moins un co-agent agrochimique, sélectionné dans le groupe constitué d'un herbicide à base de triazone sélectionné parmi l'amétridione, l'amibuzin, l'éthiozine, l'hexazinone, l'isométhiozine, le métamitron et la métribuzine, lesdites particules étant revêtues par pulvérisation avant un mélange à l'état humide, pour donner des particules au moins partiellement revêtues en surface avec un ester d'un composé sélectionné dans le groupe comprenant (a) un alcoxylate d'alkyle ou d'aryle, (b) des alcoxylates d'alcool gras, (c) des alcoxylates d'acides gras, (d) des copolymères séquencés d'alkyles, ou des oxydes d'éthylène ou des oxydes de propylène, (e) un alcoxylate aliphatique ou aromatique substitué par un polyaryle, (f) un phénol substitué par un polyaryle alcoxylé et leurs dérivés et/ou des mélanges de ceux-ci ; et (c) un manuel d'instruction comprenant des instructions pour mélanger les premier et second composants herbicides dans un rapport prédéfini et traiter les plantes, leur habitat, un champ de culture, le sol ou tout matériau autour de ceux-ci avec ledit mélange.
